(19) Europäisches Patentamt / European Patent Office / Office européen des brevets

(11) **EP 4 455 384 A1**

## (12) EUROPEAN PATENT APPLICATION
published in accordance with Art. 153(4) EPC

(43) Date of publication:
**30.10.2024 Bulletin 2024/44**

(21) Application number: **23892044.1**

(22) Date of filing: **16.11.2023**

(51) International Patent Classification (IPC):
*D04H 1/407* (2012.01)    *D04H 1/4291* (2012.01)
*D04H 1/593* (2012.01)    *D06M 15/263* (2006.01)

(52) Cooperative Patent Classification (CPC):
**D04H 1/407; D04H 1/4291; D04H 1/593; D06M 15/263**

(86) International application number:
**PCT/KR2023/018479**

(87) International publication number:
**WO 2024/106981 (23.05.2024 Gazette 2024/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **16.11.2022 KR 20220153920**
**15.11.2023 KR 20230158380**

(71) Applicant: **LG Chem, Ltd.**
**Yeongdeungpo-gu**
**Seoul 07336 (KR)**

(72) Inventors:
• **NAM, Hyemi**
**Daejeon 34122 (KR)**
• **DO, Yun Kyung**
**Daejeon 34122 (KR)**
• **NAM, Sunghun**
**Daejeon 34122 (KR)**

(74) Representative: **Plasseraud IP**
**104 Rue de Richelieu**
**CS92104**
**75080 Paris Cedex 02 (FR)**

## (54) PREPARATION METHOD FOR ABSORBENT BODY

(57) The present disclosure relates to a preparation method for an absorbent body exhibiting excellent shape retention even after moisture absorption, which can contribute to thinning the absorbent body by properly fixing the super absorbent polymer particles within the absorbent body without using pulp and can improve the filling rate by uniformly applying a super absorbent polymer.

[FIG. 1]

**Description**

[TECHNICAL FIELD]

Cross-reference to Related Application(s)

This application claims the benefit of Korean Patent Applications No. 1 0-2022-0153920 filed on November 16, 2022 and No. 10-2023-0158380 filed on November 15, 2023 with the Korean Intellectual Property Office, the disclosures of which are incorporated herein by reference in their entirety.

The present disclosure relates to a preparation method for an absorbent body.

[BACKGROUND OF ART]

In general, hygiene articles such as various diapers, sanitary pads, or incontinence pad include an absorbent body containing super absorbent polymer particles. It was common that the absorbent body mainly includes the super absorbent polymer particles and pulp to properly fix the super absorbent polymer particles while maintaining the shape of the absorbent body and hygiene articles.

However, due to the presence of the pulp, it was difficult to make the absorbent body and hygiene articles slim and thin, and there was a problem such as poor wearability in which wearer's skin against the hygiene article becomes sweaty. Moreover, since the pulp is mainly obtained from wood as a raw material, it has been contrary to the recent environmental protection trend, and the use of the pulp has become one of the main reasons of increasing manufacturing costs of the absorbent layer and sanitary articles.

For this reason, many attempts have been made to reduce the amount of pulp used in the absorbent layer and sanitary articles, or to provide sanitary articles such as so-called pulpless diapers with no pulp.

However, when the amount of pulp used was excessively reduced, many breaks in the absorbent body occurred due to low bonding strength, and furthermore, the super absorbent polymer particles were not properly fixed within the absorbent body and sanitary articles. Accordingly, the particles moved to specific parts of the absorbent body during the transportation of sanitary articles, thereby causing uneven absorption performance and preventing the sanitary article from properly maintaining its shape.

Meanwhile, in sanitary articles with no pulp such as pulpless diapers, the super absorbent polymer particles were mainly fixed using a liquid adhesive composition instead of pulp. In this way, it was attempted to achieve thinning and slimming of the absorbent body and sanitary article, and low unit cost by not using pulp. However, in conventional sanitary articles, when an excessive amount of the adhesive was used, there were disadvantages such as the deteriorated performance of the super absorbent polymer particles and the absorbent body containing the same, or process troubles occurring due to movement of the adhesive.

Conversely, when the amount of adhesive used was reduced, there was a disadvantage in that the super absorbent polymer particles were not properly fixed within the absorbent body and sanitary article and uneven absorption performance occurred due to particle movement. In addition, the problem of the shape of the absorbent body and sanitary article not being properly maintained after moisture absorption by the super absorbent polymer particles was also not solved.

Due to these problems in the prior art, there is a continuous demand for the development of the method for preparing an absorbent body that can properly fix super absorbent polymer particles in the absorbent body and sanitary articles without deteriorating performance while not using fluff pulp or using it in a significantly reduced amount, and exhibit excellent shape retention even after moisture absorption.

[DETAILED DESCRIPTION OF THE INVENTION]

[Technical Problem]

The present disclosure relates to a preparation method for an absorbent body exhibiting excellent shape retention even after moisture absorption, which can contribute to thinning the absorbent body by properly fixing the super absorbent polymer particles within the absorbent body without using pulp and can improve the filling rate by uniformly applying a super absorbent polymer.

[Technical Solution]

In order to solve the above problems, there is provided a preparation method for an absorbent body including

a first step of applying an adhesive and a super absorbent polymer on a first nonwoven fabric;

a second step of applying an adhesive on the super absorbent polymer; and

a third step of attaching a second nonwoven fabric on the first nonwoven fabric on which the second step has been completed, followed by sealing,

wherein the first nonwoven fabric and the second nonwoven fabric do not have a nap, and

the adhesive is applied without a pattern.

Meanwhile, there is also provided a preparation method for an absorbent body including

a first step of applying an adhesive and a super absorbent polymer on a first nonwoven fabric;

a second step of applying an adhesive on the super absorbent polymer; and

a third step of attaching a second nonwoven fabric on the first nonwoven fabric on which the second step has been completed, followed by sealing,

wherein the super absorbent polymer comprises

a base resin comprising a cross-linked polymer of an acrylic acid-based monomer having at least partially neutralized acidic groups and an internal cross-linking agent; and

a surface cross-linked layer formed by further cross-linking the cross-linked polymer using a surface cross-linking agent on the base resin, and

satisfies the following conditions 1) to 4):

1) a water centrifugal retention capacity (CRC) measured according to EDANA NWSP 241.0.R2 (15) is 32 g/g to 40 g/g;

2) an extractable content measured after swelling for 16 hours according to EDANA NWSP 270.0.R2 (15) is 10 wt% or less;

3) an absorption rate (vortex time) by the vortex method is 30 seconds or less; and

4) a bulk density measured according to EDANA NWSP 251.0.R2 (15) is 0.55 g/ml or more and 0.65 g/ml or less.

[ADVANTAGEOUS EFFECTS]

The absorbent body prepared according to the preparation method of the present disclosure has excellent shape retention even after moisture absorption by properly fixing the super absorbent polymer without using pulp, and also has excellent absorption performance. According to the above preparation method, a slimmer and thinner absorbent body can be manufactured without using pulp, the manufacturing costs can also be reduced, and the rewet deviation can also be reduced by fixing the super absorbent polymer.

[BRIEF DESCRIPTION OF THE DRAWINGS]

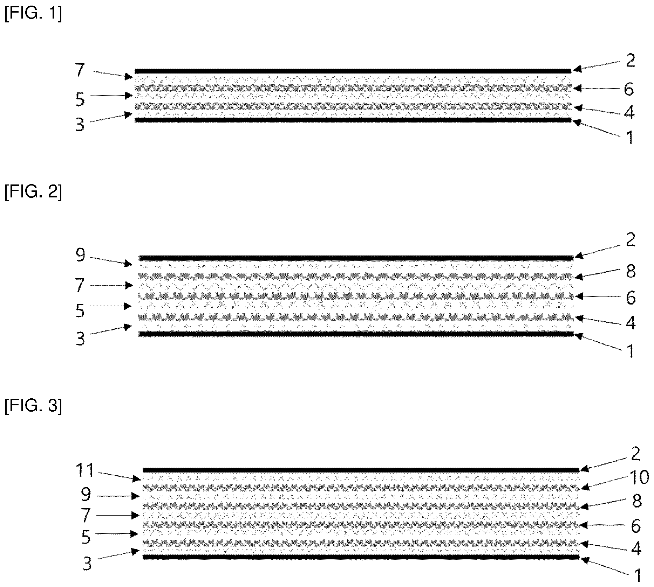

FIG. 1 is a cross-section of the absorbent body of Example 1 in which a first nonwoven fabric (1), an adhesive layer (3), a first application layer of super absorbent polymer (4), a first application layer of adhesive (5), a second application layer of super absorbent polymer (6), a second application layer of adhesive (7), and a second nonwoven fabric (2) are sequentially laminated. The above (4) to (6) are referred to as a super absorbent polymer layer.

FIG. 2 is a cross-section of the absorbent body of Example 2 in which a first nonwoven fabric (1), an adhesive layer (3), a first application layer of super absorbent polymer (4), a first application layer of adhesive (5), a second application layer of super absorbent polymer (6), a second application layer of adhesive (7), a third application layer of super absorbent polymer (8), a third application layer of adhesive (9), and a second nonwoven fabric (2) are sequentially laminated. The above (4) to (8) are referred to as a super absorbent polymer layer.

FIG. 3 is a cross-section of the absorbent body of Example 3 in which a first nonwoven fabric (1), an adhesive layer (3), a first application layer of super absorbent polymer (4), a first application layer of adhesive (5), a second application layer of super absorbent polymer (6), a second application layer of adhesive (7), a third application layer of super absorbent polymer (8), a third application layer of adhesive (9), a fourth application layer of super absorbent polymer (10), an adhesive layer (11), and a second nonwoven fabric (2) are sequentially laminated. The above (4) to (10) are referred to as a super absorbent polymer layer.

[DETAILED DESCRIPTION OF THE EMBODIMENTS]

The terminology used herein is for the purpose of describing particular embodiments only and is not intended to limit the invention. The singular forms are intended to include the plural forms as well, unless the context clearly indicates otherwise. It will be further understood that the terms "include", "have", or "possess" when used in this specification, specify the presence of stated features, steps, components, or combinations thereof, but do not preclude the presence or addition of one or more other features, steps, components, or combinations thereof.

As the present invention can be variously modified and have various forms, specific embodiments thereof are shown by way of examples and will be described in detail. However, it is not intended to limit the present invention to the particular form disclosed and it should be understood that the present invention includes all modifications, equivalents, and replacements within the idea and technical scope of the present invention.

In addition, the terminologies are used merely to refer to specific embodiments, and are not intended to restrict the present disclosure. Singular expressions of the present disclosure may include plural expressions unless they are differently expressed contextually.

In the present disclosure, the terms "the first", "the second", "the third", and the like are used to describe a variety of components, and these terms are merely employed to distinguish a certain component from other components.

The terminology "polymer" in the present disclosure is in a state in which a water-soluble ethylene-based unsaturated monomer is polymerized, and may include all moisture content ranges, or all particle diameter ranges. Among the polymers, a polymer having a moisture content of about 40 wt% or more after polymerization and before drying may be referred to as a hydrogel polymer, and particles in which the hydrogel polymer is pulverized and dried may be referred to as a cross-linked polymer.

In addition, the terminology "super absorbent polymer particle" refers to a particulate material containing a cross-linked polymer in which an acrylic acid-based monomer having at least partially neutralized acidic groups is polymerized and cross-linked by an internal cross-linking agent.

In addition, the terminology "super absorbent polymer" is used to encompass all of a cross-linked polymer in which an acrylic acid-based monomer having at least partially neutralized acidic groups is polymerized or a base resin in the form of powder consisting of super absorbent polymer particles in which the cross-linked polymer is pulverized, and the cross-linked polymer or the base resin further processed, for example, surface cross-linking, fine reassembling, drying, pulverization, classification, etc., to be in a state suitable for commercialization, depending on the context. Accordingly, the terminology "super absorbent polymer " may be interpreted as including a plurality of super absorbent polymer particles.

As the demand for thin and light diapers increases, research on pulpless diapers that do not contain pulp is underway. For pulpless diapers, the super absorbent polymer (SAP) must be able to be fixed within the absorbent body of the diaper even when the pulp is removed.

Accordingly, the present inventors studied a preparation method for an absorbent body in which an adhesive and a super absorbent polymer are sequentially applied multiple times without using pulp. In the case of the absorbent body manufactured according to this preparation method, the SAP retention rate was improved and the filling rate was also improved by uniformly applying the super absorbent polymer within the absorbent body, thereby confirming that the absorbent body had excellent shape retention and absorption performance and completing the present invention.

Hereinafter, the preparation method for an absorbent body will be described in more detail step by step according to specific embodiments of the present invention.

**(First step)**

In the first step of the preparation method for an absorbent body, an adhesive and a super absorbent polymer are applied on a first nonwoven fabric to form a super absorbent polymer layer in which the adhesive and the super absorbent polymer are alternately laminated, thereby fixing the super absorbent polymer layer having absorption performance to the nonwoven fabric.

The first nonwoven fabric has a structure without a nap. According to the preparation method according to one embodiment of the present disclosure, even without a nap, the super absorbent polymer can be fixed to the nonwoven fabric through repeated application of the adhesive and the super absorbent polymer.

Preferably, the nonwoven fabric is not particularly limited as long as it is a nonwoven fabric commonly used in absorbent body. For example, the nonwoven fabric may be a polypropylene-based nonwoven fabric, a polyethylene-based nonwoven fabric, a polyolefin-based multicomponent nonwoven fabric (e.g., a PE/PP bicomponent nonwoven layer), a nonwoven fabric made of PLA (Poly lactic acid)/PBS (Poly butyl succinate), or a non-woven fabric made of various other materials. Each of these nonwoven fabrics can be manufactured directly according to a typical nonwoven fabric manufacturing method or obtained commercially. Therefore, further explanation regarding this will be omitted.

Meanwhile, the adhesive can be applied without a specific pattern. "Applied without a pattern" means that the adhesive is applied at a substantially uniform application density over the entire application area, or the adhesive is applied randomly over the entire application area, rather than applied to a certain area. The method of fixing the super absorbent polymer by inserting an adhesive pattern into the core of the absorbent body causes an empty space inside the core due to the pattern, which lowers the filling rate of the super absorbent polymer and reduces physical properties such as retention capacity of the diapers. Meanwhile, if the adhesive is applied in the form of a network, the retention rate of the super absorbent polymer in the absorbent body may be reduced.

The adhesive may be applied according to the methods known to those skilled in the art. For example, the adhesive can be sprayed, rolled, or spun on the nonwoven fabric or super absorbent polymer surface. The type of the adhesive is also not particularly limited as long as it is an adhesive commonly used in absorbent body. For example, the adhesive may be hydrophilic, hydrophobic, biodegradable, bio-derived, or a combination thereof. Preferably, when the adhesive is applied, the super absorbent polymer layer and the nonwoven fabric exist continuously without bond sites spaced between the super absorbent polymer layer and the nonwoven fabric.

Meanwhile, a single application amount of adhesive (product name: FLC7228AZP, manufacturer: HB Fuller) on a 350 cm$^2$ nonwoven fabric is preferably 0.3 to 3 g, and the total application amount may vary depending on the number of applications. Preferably, the total amount of adhesive (product name: FLC7228AZP, manufacturer: HB Fuller) applied on a 350 cm$^2$ nonwoven fabric may be 0.3 to 10 g.

The super absorbent polymer includes a base resin including a cross-linked polymer of an acrylic acid-based monomer having at least partially neutralized acidic groups and an internal cross-linking agent; and a surface cross-linked layer formed on the base resin, wherein the cross-linked polymer is further cross-linked using a surface cross-linking agent.

The super absorbent polymer may have a water centrifugal retention capacity (CRC) of 32 g/g to 40 g/g, as measured according to EDANA NWSP 241.0.R2 (15). Preferably, the water centrifugal retention capacity of the super absorbent polymer may be 32 g/g or more, 33 g/g or more, or 33.4 g/g or more, and 40 g/g or less, 39 g/g or less, or 38.7 g/g or less. When the water centrifugal retention capacity of the super absorbent polymer is less than 32 g/g, there is a problem in that rewet performance is also reduced, and when the water retention capacity of the super absorbent polymer exceeds 40 g/g, there is a problem in manufacturing the super absorbent polymer.

The super absorbent polymer may have an extractable content of 10 wt% or less, as measured after swelling for 16 hours according to EDANA NWSP 270.0.R2 (15). Preferably, the super absorbent polymer may have an extractable content of 9 wt% or less, 8 wt% or less, or 7.7 wt% or less, and 1 wt% or more, 3 wt% or more, or 5 wt% or more, as measured after swelling for 16 hours.

The super absorbent polymer may have an absorption rate (vortex time) by the vortex method of 30 seconds or less. Preferably, it may be 29 seconds or less, or 28 seconds or less. As the lower vortex time can be evaluated as the better, the lower limit is theoretically 0 seconds, but may be about 10 seconds or more, 15 seconds or more, 18 seconds or more, 20 seconds or more, 21 seconds or more, or 22 seconds or more. When the absorption rate of the super absorbent polymer exceeds 30 seconds, there is a problem in that rewet performance is also reduced. The super absorbent polymer with an absorption rate of less than 10 seconds is not only difficult to manufacture, but also has a leakage problem because the super absorbent polymer absorbs the salt water only locally. The method of measuring the absorption rate of the super absorbent polymer will be described in more detail in Experimental Examples to be described later.

The super absorbent polymer may have a bulk density of 0.55 g/ml or more and 0.65 g/ml or less, as measured according to EDANA NWSP 251.0.R2 (15). Preferably, the bulk density of the super absorbent polymer may be 0.56 g/ml or more, 0.57 g/ml or more, or 0.58 g/ml or more, and 0.63 g/ml or less, 0.62 g/ml or less, or 0.60 g/ml or less.

Since a fast absorption rate means that the specific surface area of the particles is large, super absorbent polymer particles with a fast absorption rate have a low bulk density. If the bulk density is low, a large amount of super absorbent polymer is required, increasing manufacturing costs, so it is desirable for the bulk density to be moderately high. Considering the balance with the absorption rate, the above range of 0.55 g/ml or more and 0.65 g/ml or less is appropriate. When the bulk density is less than 0.55 g/ml, a problem of non-uniform super absorbent polymer input may occur during the manufacturing process, and when it exceeds 0.65 g/ml, flowability may be reduced.

The acrylic acid-based monomer is a compound represented by the following Chemical Formula 1:

$$[\text{Chemical Formula 1}] \qquad R^1\text{-COOM}^1$$

in Chemical Formula 1,

$R^1$ is a C2 to C5 alkyl group having an unsaturated bond, and
$M^1$ is a hydrogen atom, a monovalent or divalent metal, an ammonium group, or an organic amine salt.

Preferably, the acrylic acid-based monomer may include at least one selected from the group consisting of acrylic acid, methacrylic acid, and a monovalent metal salt, a divalent metal salt, an ammonium salt and an organic amine salt thereof.

Herein, the acrylic acid-based monomers may be those having acidic groups which are at least partially neutralized. Preferably, the acrylic acid-based monomer partially neutralized with an alkali substance such as sodium hydroxide, potassium hydroxide, ammonium hydroxide, or the like may be used. A degree of neutralization of the acrylic acid-based monomer may be 40 to 95 mol%, 40 to 80 mol %, or 45 to 75 mol%. The range of the degree of neutralization can be adjusted according to final properties. An excessively high degree of neutralization causes the neutralized monomers to be precipitated, and thus polymerization may not readily occur. On the contrary, an excessively low degree of neutralization not only deteriorates absorbency of the polymer, but also gives the polymer hard-to-handle properties, such as those of an elastic rubber.

In addition, a concentration of the acrylic acid-based monomer may be about 20 to 60 wt%, or about 40 to 50 wt% based on the monomer composition containing the raw materials of the super absorbent polymer and the solvent, and properly controlled in consideration of polymerization time and reaction conditions. When the concentration of the monomer is excessively low, the yield of the super absorbent polymer is low and there may be a problem in economic efficiency. In contrast, when the concentration is excessively high, it may cause problems in processes in that some of the monomer may be extracted or the pulverization efficiency of the polymerized hydrogel polymer may be lowered in the pulverization process, and thus physical properties of the super absorbent polymer may be deteriorated.

In addition, the terminology 'internal cross-linking agent' used herein is different from a surface cross-linking agent for cross-linking the surface of the super absorbent polymer particles to be described later, and the internal cross-linking agent polymerizes unsaturated bonds of the water-soluble ethylene-based unsaturated monomers by cross-linking. The cross-linking in the above step proceeds both on the surface and on the inside, but when the surface cross-linking process of the super absorbent polymer particles to be described later is in progress, the surface of the particles of the finally prepared super absorbent polymer has a structure cross-linked by a surface cross-linking agent, and the inside of the particles has a structure cross-linked by the internal cross-linking agent.

As the internal cross-linking agent, any compound may be used as long as it allows the introduction of cross-linking bonds during polymerization of the acrylic acid-based monomer. Specifically, the internal cross-linking agent may be a cross-linking agent having one or more ethylene-based unsaturated groups in addition to the functional group which may react with the water-soluble substituents of the acrylic acid-based monomer; or a cross-linking agent having two or more functional groups which may react with the water-soluble substituents of the monomer and/or the water-soluble substituents formed by hydrolysis of the monomer. For example, the internal cross-linking agent may be an epoxy compound.

As the internal cross-linking agent, any compound may be used as long as it allows the introduction of cross-linking bonds during polymerization of the acrylic acid-based monomer. Specifically, the internal cross-linking agent may be a cross-linking agent having one or more ethylene-based unsaturated groups in addition to the functional group which may react with the water-soluble substituents of the acrylic acid-based monomer; or a cross-linking agent having two or more functional groups which may react with the water-soluble substituents of the monomer and/or the water-soluble substituents formed by hydrolysis of the monomer.

The internal cross-linking agent may be an epoxy compound or a polyethylene glycol-based polymer. For example, as the internal cross-linking agent, a multifunctional cross-linking agent may be used alone or in combination of two or more. Examples of the internal cross-linking agent include an acrylate-based compound such as N,N'-methylenebisacrylamide, trimethylpropane tri(meth)acrylate, ethylene glycol di(meth)acrylate, polyethylene glycol (meth)acrylate, polyethylene glycol di(meth)acrylate, propylene glycol di(meth)acrylate, polypropylene glycol (meth)acrylate, butanediol di(meth)acrylate, butylene glycol di(meth)acrylate, diethylene glycol di(meth)acrylate, hexanediol di(meth)acrylate, triethylene glycol di(meth)acrylate, tripropylene glycol di(meth)acrylate, tetraethylene glycol di(meth)acrylate, dipentaerythritol pentaacrylate, glycerin tri(meth)acrylate, and pentaerythritol tetraacrylate; an epoxy-based compound such as ethylene glycol diglycidyl ether, diethylene glycol diglycidyl ether, polyethylene glycol diglycidyl ether, propylene glycol diglycidyl ether, tripropylene glycol diglycidyl ether, polypropylene glycol diglycidyl ether, neopentyl glycol diglycidyl ether, 1,6-hexanediol diglycidyl ether, polytetramethylene glycol diglycidyl ether, glycerol diglycidyl ether, glycerol triglycidyl ether, diglycerol polyglycidyl ether and polyglycerol polyglycidyl ether; triarylamine; propylene glycol; glycerin; and ethylene carbonate, but the present disclosure is not limited thereto.

According to one embodiment, the epoxy-based compound may be used as the internal cross-linking agent. For example, the internal cross-linking agent may be a divalent or higher polyvalent epoxy compound, for example, ethylene glycol diglycidyl ether. In this case, foaming by the foaming agent can be stably achieved due to the hydrophobic particles.

In the monomer composition, the internal cross-linking agent may be used in an amount of 0.01 to 5 parts by weight based on 100 parts by weight of the acrylic acid-based monomer. For example, the internal cross-linking agent may be used in an amount of 0.01 parts by weight or more, 0.05 parts by weight or more, 0.1 parts by weight, or 0.15 parts by weight or more, and 5 parts by weight or less, 3 parts by weight or less, 2 parts by weight or less, 1 part by weight or less, or 0.7 parts by weight or less based on 100 parts by weight of the water-soluble ethylene-based unsaturated monomer. When too little internal cross-linking agent is used, cross-linking does not occur sufficiently, and thus it may be difficult to achieve strength above an appropriate level, and when too much internal cross-linking agent is used, the internal cross-linking density increases, and thus it may be difficult to achieve a desired level of water retention capacity.

The cross-linking polymerization of the water-soluble ethylene-based unsaturated monomer in the presence of an internal cross-linking agent may be performed by thermal polymerization, photopolymerization or hybrid polymerization in the presence of a polymerization initiator with or without a thickener, a plasticizer, a preservation stabilizer, an antioxidant, etc., but the specific details will be described later.

Meanwhile, the super absorbent polymer further includes a surface cross-linked layer formed by further cross-linking the cross-linked polymer in the base resin using a surface cross-linking agent on at least a part of the surface of the base resin. This is to increase the surface cross-linking density of the super absorbent polymer. When the super absorbent polymer further includes a surface cross-linked layer, it has a structure having higher cross-linking density on the outside than inside.

As the surface cross-linking agent, any surface cross-linking agent that has been conventionally used in the preparation of a super absorbent polymer may be used without any particular limitation. For example, at least one selected from the group consisting of a polyalcohol-based compound, a polyepoxy-based compound, a polyamine compound, a haloepoxy compound, a condensation product of a haloepoxy compound, an oxazoline-based compound, and an alkylene carbonate-based compound may be used.

Specifically, as the polyalcohol-based compound, mono-, di-, tri-, tetra- or polyethylene glycol, monopropylene glycol, 1,3-propanediol, dipropylene glycol, 2,3,4-trimethyl-1,3-pentanediol, polypropylene glycol, glycerol, polyglycerol, 2-butene-1,4-diol, 1,4-butanediol, 1,3-butanediol, 1,5-pentanediol, 1,6-hexanediol, or 1,2-cyclohexane dimethanol may be used.

In addition, as the polyepoxy-based compound, ethylene glycol diglycidyl ether, or glycidol may be used.

As the polyamine compound, ethylenediamine, diethylenetriamine, triethylenetetraamine, tetraethylenepentamine, pentaethylenehexamine, polyethyleneimine or polyamide polyamine may be used.

Further, as the haloepoxy compound, epichlorohydrin, epibromohydrin, or α-methylepichlorohydrin may be used.

Meanwhile, as the oxazoline-based compound, mono-, di-, or polyoxazolidinone may be used.

In addition, as the alkylene carbonate-based compound, ethylene carbonate, propylene carbonate, or glycerol carbonate may be used.

More specifically, as the surface cross-linking agent, the surface cross-linking agents described above may be used alone or in combination with each other. For example, an alkylene carbonate compound such as ethylene carbonate may be used as the surface cross-linking agent.

Meanwhile, the super absorbent polymer may contain 10 wt% or less of particles having a particle diameter of 710 $\mu$m or more and 850 $\mu$m or less based on the super absorbent polymer. Additionally, the super absorbent polymer may contain 10 wt% or less of particles having a particle diameter of 150 $\mu$m or less. The particle diameter of these super absorbent polymer particles can be measured according to the EDANA WSP 220.3 (European Disposables and Nonwovens Association, EDANA).

When the content of particles having a particle diameter of 710 $\mu$m or more and 850 $\mu$m or less is high, exceeding 10 wt%, generally thin pulpless diapers may feel hard, making them less comfortable to wear, and the absorption rate may also be reduced. In addition, when the content of particles having a particle diameter of 150 $\mu$m or less exceeds 10 wt%, a problem of filter clogging may occur during the process due to a large amount of fine powder, and there is a disadvantage in that working environment becomes uncomfortable.

In addition, the super absorbent polymer may include hydrophobic particles. Herein, hydrophobic particles refer to particles having a water contact angle of 50° or more or water-insoluble particles that do not dissolve in water. Particles with a water contact angle of less than 50° and water-soluble particles may be dissolved in a monomer composition in the form of an aqueous solution, making it difficult to capture bubbles generated during the polymerization process. On the other hand, hydrophobic particles are located at the interface between the neutralizing solution and hydrophobic bubbles such as carbon dioxide in the neutralizing solution, and can effectively capture and stabilize the bubbles.

Accordingly, the hydrophobic particles have a water contact angle of 50° or more. More specifically, the hydrophobic particles may have a water contact angle of 70° or more, 100° or more, 120° or more, or 150° or more, and 175° or less.

At this time, the contact angle of the hydrophobic particles can be measured by the following method. First, a coating solution in which the hydrophobic particles are dispersed in a methylene chloride solvent at a concentration of 5 wt% is prepared. Next, this coating solution is spin-coated on a wafer without surface roughness and then dried at room temperature to remove the remaining solvent. Afterwards, water is dropped dropwise on this coating layer to measure the contact angle, which is defined as the contact angle of each hydrophobic particle.

In addition, the hydrophobic particles have an average particle diameter of 0.2 $\mu$m to 50 $\mu$m. If the average particle diameter of the hydrophobic particles is less than 0.2 $\mu$m, it is difficult to effectively collect bubbles generated during the manufacturing process, and there is a problem in that uniform pores are not formed. If the hydrophobic particles have an average particle diameter of more than 50 $\mu$m, the resulting pore size may become too large, making it difficult to improve the absorption rate of the super absorbent polymer. Specifically, the hydrophobic particles may have an average particle diameter ($\mu$m) of 0.5 or more, 1 or more, 2 or more, or 4 or more, and 40 or less, 35 or less, or 30 or less.

Herein, the average particle diameter of the hydrophobic particles means D50, and the particle diameter "Dn" means a particle diameter at the n% point of the cumulative distribution of the number of particles according to particle diameters. In other words, D50 is a particle diameter at the 50% point of the cumulative distribution of the number of particles according to particle diameters, D90 is a particle diameter at the 90% point of the cumulative distribution of the number of particles according to particle diameters, and D10 is a particle diameter at the 10% point of the cumulative distribution of the number of particles according to particle diameters. The Dn may be measured using a laser diffraction method. Specifically, the powder to be measured is dispersed in the dispersion medium and introduced into a commercially available particle size measuring device (e.g., Microtrac S3500). Then, a particle size distribution is obtained by measuring a difference in diffraction patterns according to particle diameters when the particles pass through the laser beam. In the measuring device, D10, D50 and D90 can be obtained by calculating a particle diameter at a point of reaching 10%, 50% and 90% of the cumulative distribution of the number of particles according to particle diameters.

The hydrophobic particles may be at least one selected from the group consisting of hydrophobic silica, a metal salt of a C7 to C24 fatty acid, and hydrophobic organic particles.

Herein, the hydrophobic silica is a generic term for silica having a water contact angle of 50° or more due to a small content of silanol (-SiOH) on its surface, and hydrophobic silica known in the art may be used without limitation.

In addition, the metal salt of a C7 to C24 fatty acid refers to a compound in which a metal cation is bonded instead of a hydrogen ion of a carboxyl group at the end of an unsaturated or saturated fatty acid having a linear structure while having 7 to 24 carbon atoms in the molecule, and may be a monovalent metal salt, or a polyvalent metal salt of divalent or higher. At this time, when the hydrophobic particles are a metal salt of a fatty acid having less than 7 carbon atoms, it is not possible to capture the bubbles generated in the form of particles by ionization in an aqueous solution. When the hydrophobic particles are a metal salt of a fatty acid having more than 24 carbon atoms, the chain of the fatty acid becomes long, which may cause difficult dispersion.

Specifically, when the metal salt of the fatty acid is a monovalent metal salt, it has a structure in which one fatty acid carboxylate anion is bonded to an alkali ion, which is a monovalent metal cation. In addition, when the metal salt of the fatty acid is a polyvalent metal salt of divalent or higher, it has a structure in which as many as fatty acid carboxylate anions as the number of the valence of the metal cation are bonded to the metal cation.

In one embodiment, the hydrophobic particles may be a metal salt of a C12 to C20 saturated fatty acid. For example, the hydrophobic particles may be at least one metal salt of a saturated fatty acid selected from the group consisting of a metal salt of lauric acid containing 12 carbon atoms in the molecule; a metal salt of tridecyl acid containing 13 carbon atoms in the molecule; a metal salt of myristic acid containing 14 carbon atoms in the molecule; a metal salt of pentadecanoic acid containing 15 carbon atoms in the molecule; a metal salt of palmitic acid containing 16 carbon atoms in the molecule; a metal salt of margaric acid containing 17 carbon atoms in the molecule; a metal salt of stearic acid containing 18 carbon atoms in the molecule; a metal salt of nonadecylic acid containing 19 carbon atoms in the molecule; and a metal salt of arachidic acid containing 20 carbon atoms in the molecule.

Preferably, the metal salt of a fatty acid may be a metal salt of stearic acid. For example, it may be at least one metal salt of stearic acid selected from the group consisting of calcium stearate, magnesium stearate, sodium stearate, zinc stearate and potassium stearate.

In addition, the hydrophobic organic particles may be at least one selected from the group consisting of an ethylene polymer, a propylene polymer, a styrene polymer, a butadiene polymer, a styrene-butadiene copolymer, an alkyl acrylate polymer, an alkyl methacrylate polymer, an alkyl acrylate-acrylonitrile copolymer, an acrylonitrile-butadiene copolymer, an acrylonitrile-butadiene-styrene copolymer, an acrylonitrile-alkyl acrylate-styrene copolymer, an alkyl methacrylate-butadiene-styrene copolymer, and an alkyl acrylate-alkyl methacrylate copolymer.

Meanwhile, in the first step, the adhesive and the super absorbent polymer may be applied alternately two or more times. For example, 'the application is repeated twice' means that (1) the application of an adhesive and (2) the application of a super absorbent polymer proceed in the order of (1)>(2)>(1)>(2). The number of applications is not limited, as long as they can be applied uniformly and the super absorbent polymer is fixed in the absorbent body. Preferably, the adhesive and the super absorbent polymer may be applied alternately 2 or more times, or 3 or more times, and 10 times or less, 8 times or less, 6 times or less, or 5 times or less.

At this time, the amount of super absorbent polymer used in each application of two or more application processes may be different or the same, and the amount of adhesive used in each application of two or more application processes may be different or the same. Preferably, the amount of super absorbent polymer used in each application of two or more application processes may be the same, and the amount of adhesive used in each application of two or more application processes may be the same.

Meanwhile, the super absorbent polymer does not contain pulp. Since the super absorbent polymer does not contain pulp, the mixing process with pulp is omitted in the preparation method, and a cost reduction effect can be expected.

Further, the application of the first step can preferably be performed using only the adhesive and the super absorbent polymer. Even if only the adhesive and the super absorbent polymer are applied as described above, the SAP retention rate in the absorbent body can be increased by repeated application, and the filling rate of the super absorbent polymer can also be improved by uniform application.

Preferably, a total thickness of the adhesive and the super absorbent polymer applied in the first step and the second step described later may be 5 mm or less, 4 mm or less, or 3 mm or less. The thinner the total thickness of the adhesive and the super absorbent polymer applied in the first step, the better the wearing comfort. For example, it may be 1 mm or more. If the thickness exceeds 5 mm, it becomes difficult to obtain thin and slim sanitary products.

Preferably, the super absorbent polymer applied in the first step may include 110 g or more of a super absorbent polymer per unit area ($m^2$). Preferably, it may be 110 g or more, 120 g or more, 130 g or more, 140 g or more, or 150 g or more, and 300 g or less, 250 g or less, 240 g or less, 230 g or less, 220 g or less, 210 g or less, or 200 g or less. When the super absorbent polymer content per unit area ($m^2$) of the super absorbent polymer applied in the first step is less than 100 g, the absolute amount of super absorbent polymer is insufficient, which not only deteriorates absorption performance, but also makes it difficult to maintain the shape of the absorbent core. When the super absorbent polymer content per unit area ($m^2$) exceeds 300 g, the volume of the absorbent body increases, making it difficult to thin and slim.

Meanwhile, the retention rate obtained by measuring the amount of super absorbent polymer remaining in the absorbent body without leakage after vibrating the absorbent body up and down 100 times at a frequency of 65 times/min and an amplitude of 15 mm may be 90% or more. Preferably, the retention rate may be 95% or more, 97% or more, 98% or more, 99% or more, or 99.1% or more, and 100% or less.

A filling rate may be 90% or more, wherein the filling rate is the area of a dyed part measured with a color meter after dyeing the absorbent body with a dye and drying it to confirm that only the super absorbent polymer part is selectively dyed. Preferably, the filling rate may be 95% or more, 97% or more, 98% or more, 99% or more, or 99.5% or more, and 100% or less.

**(Second step)**

When the application of the first step is completed, the last layer applied on the first nonwoven fabric (the opposite side of the first nonwoven fabric) is a super absorbent polymer, so that an adhesive is applied on the super absorbent polymer in the second step, thereby preparing an absorbent body by subsequent attachment with the second nonwoven fabric.

The adhesive used in the second step may be the same material as the adhesive in the first step.

**(Third step)**

In the third step of the preparation method for an absorbent body, the absorbent body is manufactured by attaching the second nonwoven fabric on the first nonwoven fabric to which the adhesive has been applied through the second step, followed by sealing.

The second nonwoven fabric used in the third step may be the same material as the first nonwoven fabric in the first step, and preferably, the second nonwoven fabric does not have a nap.

The application, attachment, and sealing in each step of the above preparation method can be performed according to a number of methods known to those skilled in the art, and there is no particular limitation as long as it is a commonly used method.

Hereinafter, preferred embodiments are presented to facilitate the understanding of the invention. However, the following examples are for illustrative purposes only and are not intended to limit the present disclosure.

**Preparation Example 1**

**(Step 1)** While stirring at 5000 rpm after adding 100 g of water to a high shear mixer, hydrophobic silica with an average particle diameter of 0.3 $\mu$m and a water contact angle of 130° and hydrophobic silica with an average particle diameter of 3 $\mu$m and a water contact angle of 130° were slowly added to be dispersed in an amount of 0.2 wt% and 2 wt% based on the total weight of the final aqueous dispersion, respectively. When the silica was completely added, stirring was performed at 8000 rpm for 30 minutes at a temperature of 45 °C. At this time, the pH of the obtained aqueous dispersion of hydrophobic silica was 9.

**(Step 2)** A monomer solution was prepared by mixing 100 parts by weight of acrylic acid, 0.01 parts by weight of PEGDA 400 (polyethylene glycol diacrylate 400) as an internal cross-linking agent, and 0.1 part by weight of phenyl-

bis(2,4,6-trimethylbenzoyl)phosphine oxide as a photoinitiator. Subsequently, a monomer composition was prepared by continuously adding 160 parts by weight of a 24 wt% aqueous solution of sodium hydroxide while continuously supplying the monomer solution using a metering pump, and then high-speed mixing 3 parts by weight of 4 wt% aqueous solution of sodium persulfate, 5 parts by weight of 4 wt% ethylene glycol diglycidyl ether (EJ1030s), and 5 parts by weight of 4 wt% aqueous solution of sodium bicarbonate, and 5 parts by weight of the aqueous dispersion of hydrophobic silica prepared in Step 1 containing 0.2 wt% of hydrophobic silica with an average particle diameter of 0.3 $\mu$m and 2 wt% of hydrophobic silica with an average particle diameter of 3 $\mu$m. Through this transfer, the monomer composition was added to a polymerization reactor including a moving conveyor belt, and UV polymerization was performed for 3 minutes by irradiating ultraviolet rays through a UV irradiation device to prepare a sheet-type hydrogel polymer.

(Step 3) The hydrogel polymer was cut to an average size of about 300 mm or less, and then placed in a pulverizing machine (equipped with a perforated plate containing a plurality of holes with a diameter of 15 mm) to pulverize the hydrogel. Thereafter, the pulverized hydrogel was dried in a dryer capable of changing wind direction up and down. The hydrogel was dried uniformly by flowing hot air at 180 °C so that the moisture content of the dried powder was about 5% or less.

(Step 4) The dried polymer was pulverized with a pulverizing machine and then classified to obtain a base resin with a size of 150 to 850 $\mu$m. The used pulverizing machine was PULVERISETTE 19 from FRITSCH. A 12 mm sieve cassette was installed in the pulverizing machine, and the dried hydrogel was first pulverized. Among the sieves (W.S. Tylor, 8" STAINLESS - STAINLESS TEST SIEVE) below, the sieves with mesh number of 24/32/48/100/pan were installed sequentially. Then, the pulverized particles were added to the top of the #24 mesh, fixed on a sieve shaker, and classified at 1.5 amplitude for 10 minutes.

8" STAINLESS - STAINLESS TEST SIEVES.

| Catalog Number | Description | Mesh | Microns |
|---|---|---|---|
| 040A-805-20 | 8"-FH-SS-SS-US-20 | 20 | 850 |
| 040A-805-25 | 8"-FH-SS-SS-UA-25 | 24 | 710 |
| 040A-805-35 | 8"-FH-SS-SS-US-35 | 32 | 500 |
| 040A-805-50 | 8"-FH-SS-SS-US-50 | 48 | 300 |
| 040A-805-100 | 8"-FH-SS-SS-US-100 | 100 | 150 |
| 040A-805-170 | 8"-FH-SS-SS-US-170 | 170 | 90 |
| 040A-805-325 | 8"-FH-SS-SS-US-325 | 325 | 45 |

After the classification was completed, the polymer content at the top of the #24 mesh was measured, and 50 wt% or more of particles were measured compared to the total amount of dried hydrogel added.

The particles at the top of the #24 mesh were subjected to second pulverization. In the second pulverization, a 1.0 mm sieve cassette was installed in the pulverizing machine and the dried hydrogel was pulverized. Among the sieves (W.S. Tylor, 8" STAINLESS - STAINLESS TEST SIEVE), the sieves with mesh number of 20/32/48/100/pan were installed sequentially. Then, the particles that had undergone the second pulverization were added to the top of the #20 mesh, fixed on a sieve shaker, and classified at 1.5 amplitude for 10 minutes.

The particle weight after classification can be obtained as follows:

Particle size distribution according to particle size = {(sieve weight + dried body after classification) - (empty sieve weight)} /(dried body after pulverization) *100

(Step 5) 8 parts by weight of an aqueous surface cross-linking solution containing 1.2 parts by weight of ethylene carbonate, 0.5 parts by weight of propylene carbonate, 0.5 parts by weight of propylene glycol, and 0.5 parts by weight of hydrophilic water-dispersed silica (snotex) was sprayed onto 100 parts by weight of the base resin powder obtained by classification, and stirred at room temperature to ensure that the surface cross-linking solution was evenly distributed on the base resin powder. Thereafter, the base resin powder mixed with the surface cross-linking solution was added to a surface cross-linking reactor and a surface cross-linking reaction was performed.

In this surface cross-linking reactor, the temperature of the base resin powder was gradually increased from an initial temperature around 80 °C, and it was operated to reach a maximum reaction temperature of 190 °C after 30 minutes. After reaching this maximum reaction temperature, the reaction was further performed for 15 minutes and a sample of the finally produced super absorbent polymer was taken. After the surface cross-linking process, the super absorbent polymer of Example 1 having a particle size of 150 $\mu$m to 850 $\mu$m was prepared by classification with a ASTM standard mesh.

**Example 1**

After uniformly applying 0.4 g of an adhesive (product name: FLC7228AZP, manufacturer: HB Fuller) (adhesive layer, 3) on a first nonwoven fabric (product name: Softhann®, manufacturer: Sambo) (1) of 350 mm * 100 mm in size with a hot melt sprayer, 6 g of the super absorbent polymer prepared in Preparation Example was uniformly applied at a feed rate of 2 g/s to prepare a first application layer (4) of the super absorbent polymer. 0.4 g of the adhesive was applied thereon in the same manner to prepare a first application layer (5) of the adhesive. Thereafter, the process of preparing the first application layer (4) of the super absorbent polymer and the first application layer (5) of the adhesive was repeated one more time to produce a second application layer (6) of the super absorbent polymer and an adhesive layer (second application layer of adhesive, 7). Then, an absorbent body was manufactured by attaching a second nonwoven fabric (product name: Softhann®, manufacturer: Sambo) (2). The amount of adhesive used in each adhesive layer was the same as 0.4 g, and the amount of super absorbent polymer used in each super absorbent polymer layer was the same as 6 g.

**Example 2**

After uniformly applying 0.3 g of an adhesive (product name: FLC7228AZP, manufacturer: HB Fuller) (adhesive layer, 3) on a first nonwoven fabric (product name: Softhann®, manufacturer: Sambo) (1) of 350 mm * 100 mm in size with a hot melt sprayer, 4 g of the super absorbent polymer prepared in Preparation Example was uniformly applied at a feed rate of 2 g/s to prepare a first application layer (4) of the super absorbent polymer. 0.3 g of the adhesive was applied thereon in the same manner to prepare a first application layer (5) of the adhesive. Thereafter, the process of preparing the first application layer (4) of the super absorbent polymer and the first application layer (5) of the adhesive was repeated two more times to produce a second application layer (6) of the super absorbent polymer, a second application layer (7) of the adhesive, a third application layer (8) of the super absorbent polymer, and an adhesive layer (third application layer of adhesive, 9). Then, an absorbent body was manufactured by attaching a second nonwoven fabric (product name: Softhann®, manufacturer: Sambo) (2). The amount of adhesive used in each adhesive layer was the same as 0.3 g, and the amount of super absorbent polymer used in each super absorbent polymer layer was the same as 4 g.

**Example 3**

After uniformly applying 0.24 g of an adhesive (product name: FLC7228AZP, manufacturer: HB Fuller) (adhesive layer, 3) on a first nonwoven fabric (product name: Softhann®, manufacturer: Sambo) (1) of 350 mm * 100 mm in size with a hot melt sprayer, 3 g of the super absorbent polymer prepared in Preparation Example was uniformly applied at a feed rate of 2 g/s to prepare a first application layer (4) of the super absorbent polymer. 0.24 g of the adhesive was applied thereon in the same manner to prepare a first application layer (5) of the adhesive. Thereafter, the process of preparing the first application layer (4) of the super absorbent polymer and the first application layer (5) of the adhesive was repeated three more times to produce a second application layer (6) of the super absorbent polymer, a second application layer (7) of the adhesive, a third application layer (8) of the super absorbent polymer, a third application layer (9) of the adhesive, a fourth application layer (10) of the super absorbent polymer, and an adhesive layer (11). Then, an absorbent body was manufactured by attaching a second nonwoven fabric (product name: Softhann®, manufacturer: Sambo) (2). The amount of adhesive used in each adhesive layer was the same as 0.24 g, and the amount of super absorbent polymer used in each super absorbent polymer layer was the same as 3 g.

**Comparative Example 1**

A mixture in which 12 g of the super absorbent polymer of Preparation Example 1 and 5 g of pulp were mixed uniformly with air in a chamber was evenly applied to the center of a first nonwoven fabric (product name: Softhann®, manufacturer: Sambo) (1) of 350 mm * 100 mm in size. Then, 1.2 g of an adhesive was uniformly applied to the edge excluding the portion where the super absorbent polymer and pulp mixture were placed on the first nonwoven fabric with a hot melt sprayer, and then a second nonwoven fabric was attached thereto to prepare an absorbent body.

**Comparative Example 2**

After uniformly applying 0.6 g of an adhesive (product name: FLC7228AZP, manufacturer: HB Fuller) (adhesive layer) on a first nonwoven fabric (product name: Softhann®, manufacturer: Sambo) of 350 mm * 100 mm in size with a hot melt sprayer, 12 g of the super absorbent polymer prepared in Preparation Example was uniformly applied thereto at a feed rate of 2 g/s to prepare a super absorbent polymer application layer. Then, 0.6 g of an adhesive was applied thereon in the same manner to prepare an adhesive layer, and then a second nonwoven fabric (product name: Softhann®, manufacturer: Sambo) was attached thereto to prepare an absorbent body.

**Experiment Examples**

For the absorbent bodies prepared in Examples 1 to 3 and Comparative Examples 1 and 2, a retention rate and rewet performance of the super absorbent polymer in the absorbent bodies were measured and are shown in Table 1 below.

(1) SAP retention rate (%) in absorbent body

The SAP retention rate in nonwoven fabric is tested by measuring the leakage of core super absorbent polymer in the nonwoven fabric. The amount of super absorbent polymer remaining in the absorbent body without leakage after vibrating each absorbent body of Examples and Comparative Examples was measured, and represented as a percentage. The vibration was performed by fixing a part 1 cm away from the top of the absorbent body to a vibration device (device name: BLM512OHP-100S, manufacturer: oriental motor), and then vibrating it 100 times in the vertical direction at a frequency of 65 times/min and an amplitude of 15 mm.

The retention rate was calculated as $(W1/WO)*100$, where WO is the weight of the absorbent body before vibration and W1 is the weight of the absorbent body after vibration. This allows us to know the degree of SAP departure within the absorbent body due to vibration, so the above value is used as an indicator to check how much SAP is fixed in the absorbent body.

(2) SAP filling rate (%)

The SAP filling rate refers to the area ratio occupied by the super absorbent polymer within the absorbent body, and is measured as follows:

The absorbent body was dyed with a dye (product name: FD&C BLUE NO. 1 POWDER, manufacturer: SENSIENT) and dried. After confirming that only the super absorbent polymer part was selectively dyed, the area of the dyed part was measured with a color meter (device name: Labscan XE, manufacturer: Hunter lab) under the conditions of a spectral range of 400 nm to 700 nm, and a wavelength resolution of < 3 nm.

(3) Rewet performance (Rewet, g)

The rewet performance under no pressure/pressure conditions of the absorbent bodies of Examples and Comparative Examples was evaluated by the following method.

85 mL of a 0.9 wt% sodium chloride aqueous solution (physiological saline) was injected into the center of each absorbent body (350 mm * 100 mm * 3 mm in size), and 2 minutes later, a paper (filter paper of 300 gsm (S-300, Hankuk Paper), approximately 1.5 $g(W_6(g))$/sheet) of 300 mm * 90 mm in size was placed thereon. 2 minutes later, the amount of physiological saline emerged from the absorbent body to the paper was measured and the paper was removed. 15 minutes after the injection of the physiological saline, a weight was placed on the absorbent body and an additional 85 mL of physiological saline was injected into the center of the absorbent body at the same location while applying a pressure of 0.42 psi. 15 minutes later, the weight on the absorbent body was temporarily removed and a new paper (filter paper of 300 gsm (S-300, Hankuk Paper), approximately 1.5 $g(W_6(g))$/sheet) was placed on the absorbent body. Then, the weight was placed back on the paper to position the paper between the absorbent body and the weight. 2 minutes later, the amount of physiological saline emerged from the absorbent body to the paper was measured and the rewet (g) was calculated according to the following Equation 1.

[Equation 1]

$$Rewet\ (g) = (W_4(g) - W_5(g)) + (W_6(g) - W_5(g))$$

In Equation 1,
W$_4$ (g) is a weight of the paper that absorbed the liquid emerged from the absorbent body for 2 minutes, 2 minutes after injecting physiological saline into the absorbent body under no pressure, W$_5$ (g) is an initial weight of the paper, and W$_6$ (g) is a weight of the paper that absorbed the liquid emerged from the absorbent body for 2 minutes at a load of 0.42 psi after injecting physiological saline into the absorbent body under no pressure and under pressure.

[Table 1]

| | Number of applications of SAP+adhesive (n) | SAP Retention rate in absorbent body (wt%, SAP Retention on pad Test) | SAP filling rate (%) | Total thickness of adhesive layer and super absorbent polymer layer (mm) | Rewet (g) |
|---|---|---|---|---|---|
| Comp. Ex. 1 | . | 60 | 70 | 10 | 6.5 |
| Comp. Ex. 2 | 1 | 80 | 100 | ≤5 | 4.3 |
| Ex. 1 | 2 | 99 | 100 | ≤5 | 2.6 |
| Ex. 2 | 3 | 99.50 | 100 | ≤5 | 2.4 |
| Ex. 3 | 4 | 99.20 | 100 | ≤5 | 2.3 |

Referring to Table 1, the same amount of adhesive and the same amount of super absorbent polymer were used in Comparative Examples and Examples 1 to 3. However, it was confirmed that when the number of applications was 2 or more, the SAP retention rate in the absorbent body was very high as more than 99%, and the rewet performance was also excellent.

[DESCRIPTION OF SYMBOLS]

1: First nonwoven fabric
2: Second nonwoven fabric
3: Adhesive layer
4: First application layer of super absorbent polymer
5: First application layer of adhesive
6: Second application layer of super absorbent polymer
7: Second application layer of adhesive
8: Third application layer of super absorbent polymer
9: Third application layer of adhesive
10: Fourth application layer of super absorbent polymer
11: Adhesive layer

**Claims**

1.  A preparation method for an absorbent body comprising

    a first step of applying an adhesive and a super absorbent polymer on a first nonwoven fabric;
    a second step of applying an adhesive on the super absorbent polymer; and
    a third step of attaching a second nonwoven fabric on the first nonwoven fabric on which the second step has been completed, followed by sealing,
    wherein the application of the adhesive and the super absorbent polymer is alternately repeated two or more times in the first step,
    the first nonwoven fabric and the second nonwoven fabric do not have a nap, and
    the adhesive is applied without a pattern.

2.  The preparation method for an absorbent body of Claim 1,
    wherein the super absorbent polymer does not contain pulp.

3. The preparation method for an absorbent body of Claim 1,
wherein a total thickness of the adhesive and the super absorbent polymer applied in the first and second steps is 5 mm or less.

4. The preparation method for an absorbent body of Claim 1,
wherein the super absorbent polymer applied in the first step comprises 100 g or more of a super absorbent polymer per unit area ($m^2$).

5. The preparation method for an absorbent body of Claim 1,
wherein the application in the first step is performed only with the adhesive and the super absorbent polymer.

6. The preparation method for an absorbent body of Claim 1,

wherein the absorbent body has a retention rate of 90% or more, wherein the retention rate is obtained by measuring an amount of super absorbent polymer remaining in the absorbent body without leakage after vibrating the absorbent body up and down 100 times at a frequency of 65 times/min and an amplitude of 15 mm, and a filling rate of 90% or more, wherein the filling rate is an area of a dyed part measured with a color meter after dyeing the absorbent body with a dye and drying it to confirm that only a super absorbent polymer part is selectively dyed.

7. The preparation method for an absorbent body of Claim 1,

wherein the super absorbent polymer comprises
a base resin comprising a cross-linked polymer of an acrylic acid-based monomer having at least partially neutralized acidic groups and an internal cross-linking agent; and
a surface cross-linked layer formed by further cross-linking the cross-linked polymer using a surface cross-linking agent on the base resin, and
satisfies the following conditions 1) to 4):

1) a water centrifugal retention capacity (CRC) measured according to EDANA NWSP 241.0.R2 (15) is 32 g/g to 40 g/g;
2) an extractable content measured after swelling for 16 hours according to EDANA NWSP 270.0.R2 (15) is 10 wt% or less;
3) an absorption rate, i.e. vortex time, by the vortex method is 30 seconds or less; and
4) a bulk density measured according to EDANA NWSP 251.0.R2 (15) is 0.55 g/ml or more and 0.65 g/ml or less.

8. A preparation method for an absorbent body comprising

a first step of applying an adhesive and a super absorbent polymer on a first nonwoven fabric;
a second step of applying an adhesive on the super absorbent polymer; and
a third step of attaching a second nonwoven fabric on the first nonwoven fabric on which the second step has been completed, followed by sealing,
wherein the super absorbent polymer comprises
a base resin comprising a cross-linked polymer of an acrylic acid-based monomer having at least partially neutralized acidic groups and an internal cross-linking agent; and
a surface cross-linked layer formed by further cross-linking the cross-linked polymer using a surface cross-linking agent on the base resin, and
satisfies the following conditions 1) to 4):

1) a water centrifugal retention capacity (CRC) measured according to EDANA NWSP 241.0.R2 (15) is 32 g/g to 40 g/g;
2) an extractable content measured after swelling for 16 hours according to EDANA NWSP 270.0.R2 (15) is 10 wt% or less;
3) an absorption rate, i.e. vortex time, by the vortex method is 30 seconds or less; and
4) a bulk density measured according to EDANA NWSP 251.0.R2 (15) is 0.55 g/ml or more and 0.65 g/ml or less.

EP 4 455 384 A1

[FIG. 1]

7 — 2
5 — 6
3 — 4
— 1

[FIG. 2]

9 — 2
7 — 8
5 — 6
3 — 4
— 1

[FIG. 3]

11 — 2
9 — 10
7 — 8
5 — 6
3 — 4
— 1

15

<div align="center">

**INTERNATIONAL SEARCH REPORT**

</div>

| | International application No. |
|---|---|
| | **PCT/KR2023/018479** |

**A.    CLASSIFICATION OF SUBJECT MATTER**

**D04H 1/407**(2012.01)i; **D04H 1/4291**(2012.01)i; **D04H 1/593**(2012.01)i; **D06M 15/263**(2006.01)i

According to International Patent Classification (IPC) or to both national classification and IPC

**B.    FIELDS SEARCHED**

Minimum documentation searched (classification system followed by classification symbols)

D04H 1/407(2012.01); A61F 13/45(2006.01); A61F 13/53(2006.01); A61F 13/534(2006.01); A61F 13/535(2006.01); A61F 13/539(2006.01); B32B 5/26(2006.01); C08J 3/12(2006.01); C08J 3/24(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 흡수체(absorber), 부직포(non-woven fabric), 고흡수성 수지(super absorbent polymer), 접착제(adhesive), 기모(napping), 펄프(pulp), 고정율(fitting ratio), 충진율(fill-factor)

**C.    DOCUMENTS CONSIDERED TO BE RELEVANT**

| Category* | Citation of document, with indication, where appropriate, of the relevant passages | Relevant to claim No. |
|---|---|---|
| X | JP 2019-093194 A (PROCTER & GAMBLE COMPANY) 20 June 2019 (2019-06-20)<br>See paragraphs [0017], [0032]-[0044], [0082]-[0093], [0103]-[0104] and [0111]-[0123]; and figure 7. | 1-8 |
| A | JP 2020-137685 A (NIPPON PAPER CRECIA CO., LTD.) 03 September 2020 (2020-09-03)<br>See entire document. | 1-8 |
| A | KR 10-2019-0087208 A (LG CHEM, LTD.) 24 July 2019 (2019-07-24)<br>See entire document. | 1-8 |
| A | JP 2000-238161 A (TOYO EIZAI CORP.) 05 September 2000 (2000-09-05)<br>See entire document. | 1-8 |
| A | JP 2022-041041 A (NIPPON PAPER CRECIA CO., LTD.) 11 March 2022 (2022-03-11)<br>See entire document. | 1-8 |

☐ Further documents are listed in the continuation of Box C.     ☑ See patent family annex.

| | |
|---|---|
| *       Special categories of cited documents:<br>"A"    document defining the general state of the art which is not considered to be of particular relevance<br>"D"    document cited by the applicant in the international application<br>"E"    earlier application or patent but published on or after the international filing date<br>"L"    document which may throw doubts on priority claim(s) or which is cited to establish the publication date of another citation or other special reason (as specified)<br>"O"    document referring to an oral disclosure, use, exhibition or other means<br>"P"    document published prior to the international filing date but later than the priority date claimed | "T"    later document published after the international filing date or priority date and not in conflict with the application but cited to understand the principle or theory underlying the invention<br>"X"    document of particular relevance; the claimed invention cannot be considered novel or cannot be considered to involve an inventive step when the document is taken alone<br>"Y"    document of particular relevance; the claimed invention cannot be considered to involve an inventive step when the document is combined with one or more other such documents, such combination being obvious to a person skilled in the art<br>"&"    document member of the same patent family |

| Date of the actual completion of the international search | Date of mailing of the international search report |
|---|---|
| **20 February 2024** | **20 February 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2023/018479**

| Patent document cited in search report | | | Publication date (day/month/year) | Patent family member(s) | | | Publication date (day/month/year) |
|---|---|---|---|---|---|---|---|
| JP | 2019-093194 | A | 20 June 2019 | CA | 2838951 | A1 | 13 December 2012 |
| | | | | CA | 2838951 | C | 16 July 2019 |
| | | | | CA | 3042501 | A1 | 13 December 2012 |
| | | | | CA | 3042501 | C | 23 June 2020 |
| | | | | CL | 2013003519 | A1 | 11 July 2014 |
| | | | | CN | 103596534 | A | 19 February 2014 |
| | | | | CN | 103596534 | B | 12 April 2017 |
| | | | | CN | 105816277 | A | 03 August 2016 |
| | | | | DE | 202012013585 | U1 | 17 January 2018 |
| | | | | EP | 2717821 | A1 | 16 April 2014 |
| | | | | EP | 2717821 | B1 | 07 August 2019 |
| | | | | EP | 3266430 | A1 | 10 January 2018 |
| | | | | EP | 3266430 | B1 | 01 July 2020 |
| | | | | EP | 3284449 | A1 | 21 February 2018 |
| | | | | EP | 3284449 | B1 | 02 October 2019 |
| | | | | JP | 2014-515983 | A | 07 July 2014 |
| | | | | JP | 2017-124188 | A | 20 July 2017 |
| | | | | JP | 6797954 | B2 | 09 December 2020 |
| | | | | US | 10149788 | B2 | 11 December 2018 |
| | | | | US | 10517777 | B2 | 31 December 2019 |
| | | | | US | 10893987 | B2 | 19 January 2021 |
| | | | | US | 2012-0316528 | A1 | 13 December 2012 |
| | | | | US | 2015-0038931 | A1 | 05 February 2015 |
| | | | | US | 2018-0207039 | A1 | 26 July 2018 |
| | | | | US | 2019-0365578 | A1 | 05 December 2019 |
| | | | | US | 2019-0365579 | A1 | 05 December 2019 |
| | | | | WO | 2012-170781 | A1 | 13 December 2012 |
| | | | | ZA | 201400178 | B | 27 January 2016 |
| JP | 2020-137685 | A | 03 September 2020 | | None | | |
| KR | 10-2019-0087208 | A | 24 July 2019 | KR | 10-2577709 | B1 | 11 September 2023 |
| JP | 2000-238161 | A | 05 September 2000 | JP | 3908403 | B2 | 25 April 2007 |
| JP | 2022-041041 | A | 11 March 2022 | | None | | |

Form PCT/ISA/210 (patent family annex) (July 2022)

**REFERENCES CITED IN THE DESCRIPTION**

**Patent documents cited in the description**

- KR 1020220153920

- KR 1020230158380